# EUROPEAN PATENT SPECIFICATION

(11) **EP 1 924 220 B1**
(45) Date of publication and mention of the grant of the patent: **03.08.2011**
(21) Application number: 06813733.0
(22) Date of filing: 23.08.2006
(51) Int. Cl.: A61F 2/06, A61F 2/84

(54) **ENDOLUMINAL PROSTHESIS ADAPTED TO DEPLOYMENT IN A DISTORTED BRANCHED BODY LUMEN**
ENDOLUMINALE PROTHESE ZUR ANWENDUNG IN EINEM GESTÖRTEN, VERZWEIGTEN KÖRPERLUMEN
PROTHESE ENDOLUMINALE CONVENANT POUR LA POSE DANS UNE LUMIERE CORPORELLE RAMIFIEE DEFORMEE

(30) Priority: 25.08.2005 US 211895
(43) Date of publication of application: 28.05.2008
(73) Proprietor: Boston Scientific Limited, Hastings Christ Church (BB)
(72) Inventor: O'BRIEN, Mark, E., Natick, MA 01760 (US); SHERRY, John, E., Needham, MA 02492-4243 (US)
(74) Representative: Hauck Patent- und Rechtsanwälte
(86) International application number: PCT/US2006/033174
(87) International publication number: WO 2007/025101

(56) References cited:
- EP-A- 1 498 085
- US-A- 5 824 040
- US-A1- 2003 120 333

## Description

### BACKGROUND OF THE INVENTION

It is known to treat an abdominal aortic aneurysm (AAA) by endoluminally implanting a bifurcated stent-graft in order to bypass the aneurysm. Stent-grafts adapted for this use include a number of designs. A large class of these designs incorporates a modular aspect that brings the benefits of reducing the size of each individual subcomponent to be implanted and facilitating a customized fit for each patient. A downside of modular design is that it necessitates in situ assembly of the sub-components. A common in situ assembly process of a two-piece device includes an initial insertion of a bifurcated component whose branch portions are of differing lengths; so called "long leg - short leg devices." The bifurcated component is inserted from a remote location into and up an ipsilateral iliac artery and positioned in the aortic neck so that the second branch portion directs luminal flow into the contralateral iliac artery. Next, a leg extension component is engaged with the second branch portion to extend the bifurcated device into the contralateral iliac artery. This can be achieved by inserting a guidewire from a remote location into the contralateral iliac artery and through the aperture of the second branch portion. The leg extension component is then delivered along the guidewire and into engagement with the second branch portion of the bifurcated component.

In patients with large saccular aneurysms, the aortic neck is typically angularly offset significantly from the iliac arteries. The offset hinders insertion of the leg extension component guidewire into the second branch portion aperture because the guidewire approaches the aperture from an acute angle.

An endovascular prosthesis including unbranched portion and at least two branch portions has become known from US 2003/0120333 A1 and US 5,824,040 A.

### SUMMARY OF THE INVENTION

The present invention relates to a prosthetic device for facilitating endoluminal assembly of a modular bifurcated endoluminal device in a branched body lumen from a remote location according to claim 1. The invention is particularly advantageous in the treatment of large saccular AAA's with a distorted aortic neck.

Generally, an endoluminal device is adapted to be deployed in a branched body lumen, from an access location remote from the location at which the device is to be deployed, by introduction through one of the branches of the branched body lumen. The device comprises an unbranched portion and at least two branch portions in communication with said unbranched portion. The branch portions comprise a first branch portion adapted to be deployed in one branch of the body lumen and a second branch portion adapted to be directed toward a second branch of the body lumen. The device further comprises a distortion element removeably connected to the second branch portion for manipulating the geometry of the second branch portion. The distortion element extends along the first branch portion to the access location. The distortion element can be an elongated filament such as a wire, a yarn, or a cable. The removable connection can be a slipknot, a ring, or an eyelet. Optionally, the present invention also includes: a second endoluminal device adapted to be mated with the second branch portion of the first device; the methods for using and inserting those devices, and apparatus therefore.

### BRIEF DESCRIPTION OF THE DRAWINGS

The invention is best understood from the following detailed description when read in connection with the accompanying drawings. It is emphasized that, according to common practice, the various features of the drawings are not to scale. On the contrary, the dimensions of the various features may be arbitrarily expanded or reduced for clarity. Included in the drawings are the following Figures:
Figure 1 illustrates a typical large saccular aneurysm having an offset neck;
Figure 2A illustrates a prior art bifurcated endoluminal device having a long leg and a second branch portion;
Figure 2B illustrates use of the prior art device depicted in Figure 2A in a distorted aneurysm;
Figure 3A illustrates a first device not according to the present invention;
Figure 3B illustrates the device of Figure 3A in use in a distorted aneurysm;
Figure 3C illustrates the device of figure 3A in use in a distorted aneurysm showing attachment of an optional leg extension;
Figure 4A illustrates a device embodiment of the present invention;
Figure 4B illustrates the device of Figure 4A in use in a distorted aneurysm;
Figure 4C illustrates the device of figure 4A in use in a distorted aneurysm showing attachment of an optional leg extension;
Figure 5A illustrates an introducer showing a loaded endoluminal device fully collapsed within the introducer;
Figure 5B illustrates the introducer of Figure 5A with the loaded endoluminal device partially deployed;
Figure 5C illustrates the introducer of Figure 5A with the loaded endoluminal device deployed;
Figures 6A-C are cross-sectional views of the loaded introducer shown in Figure 5A at the respective cross-section planes 6A-6A, 6B-6B, and 6C-6C;
Figure 7 illustrates an exemplary second introducer for deployment of as leg-extending second device;
Figure 8A illustrates a device including an everted leg in use in a distorted aneurysm; and
Figure 8B illustrates the everted leg device of Figure 8A with the everted leg deployed.

### DETAILED DESCRIPTION OF THE INVENTION

The invention will next be illustrated with reference to the figures wherein the same numbers indicate similar elements in all figures. Such figures are intended to be illustrative rather than limiting and are included herewith to facilitate the explanation of the apparatus of the present invention.

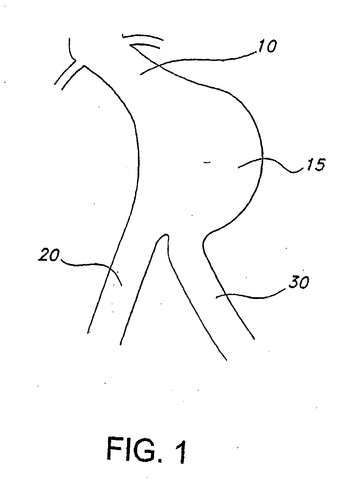

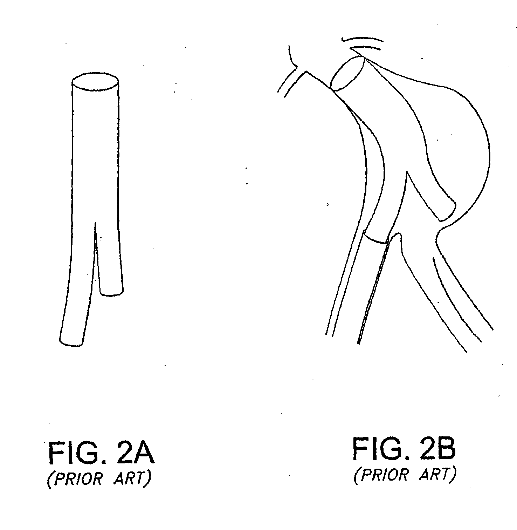

Figure 1 illustrates an exemplary AAA having a large saccular portion 15 and a tortured neck 10 distorted from its natural vertical alignment. Ipsilateral iliac artery 20 and contralateral iliac artery 30 are also indicated. Figure 2A depicts a bifurcated "long leg - second branch portion" prosthetic device used in the prior art to treat AAA's. Figure 2B illustrates the difficulties in an exemplary prior art device being implanted in a large saccular AAA having a tortured neck.

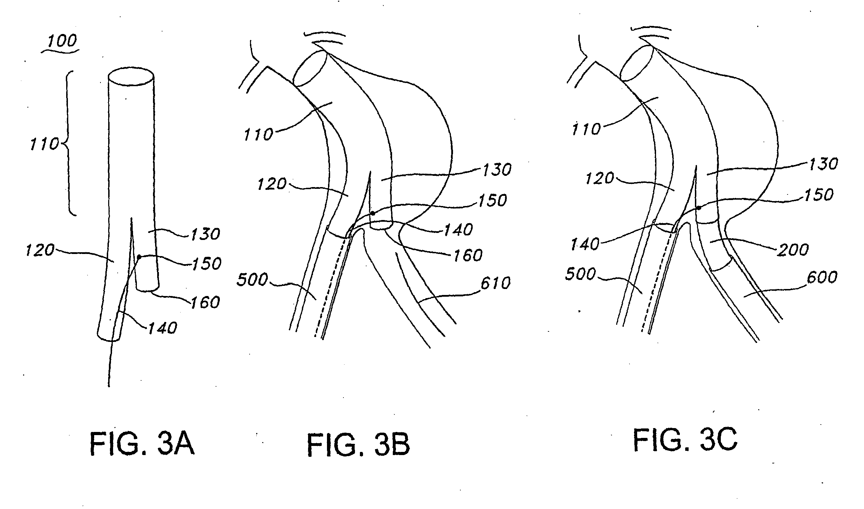

In Figure 3A is shown a bifurcated endoluminal device 100 that includes a distortion element 140 removeably connected to second branch portion 130. Distortion element 140 can take the form of any elongated filament that is capable of translating a pulling force from a remote location to the second branch portion 130. Examples of a suitable elongated filament are a wire, yarn, or cable. Distortion element 140 is connected to second branch portion 130 by a removable connection means 150 such as a slipknot, ring, or eyelet by which distortion element 140 can be detached following its use.

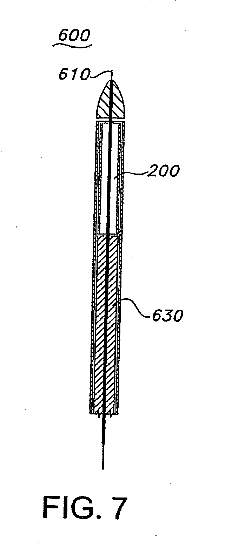

Figure 3B shows the device in usewhereby distortion element 140 enables the surgeon to effectively pull second branch portion 130 toward first branch portion 120 by applying tension or otherwise manipulating distortion element 140. Thus, the surgeon is able to facilitate the location of open end 160 of second branch portion 130 with guidewire 610. Using guidewire 610, the surgeon can optionally deploy and mate a second device (200) to second branch portion (130) using second introducer 600 (an example of which is illustrated at Figure 7) as needed to extend second branch portion (130) as shown in Figure 3C. When distortion element 140 is no longer needed it can be detached from second branch portion 130 by detaching removeable connection means 150 and pulling it through the ipsiliateral iliac 30. Preferably, removal is performed through introducer 500 (discussed further below) by which device 100 was originally introduced. For example, where connection means 150 is a slipknot, simply pulling on the slipknot to untie it and then pulling distortion element 140 through introducer 500. Likewise, where connection means 150 is a slip ring, pulling one end of distortion element 140 through the slip ring and then pulling distortion element 140 through introducer 500.

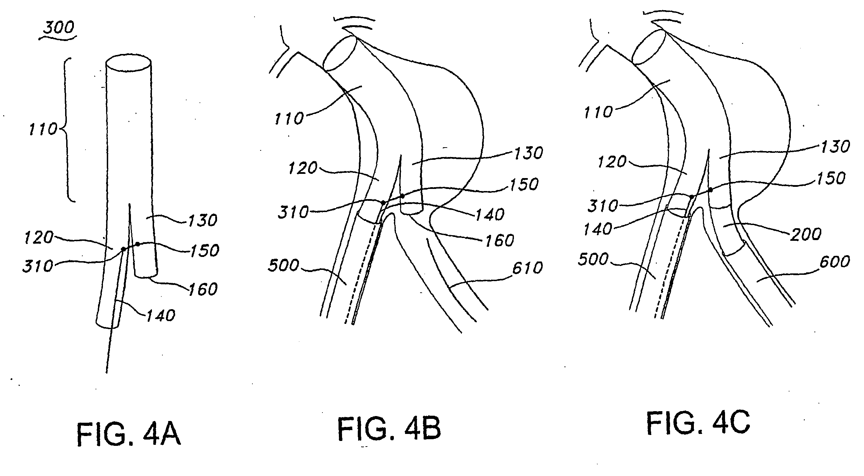

As proposed herein and as shown in Figure 4A, an embodiment of the present invention is a bifurcated endoluminal device 300. Bifurcated device 300 includes the features of bifurcated device 100 and further incorporates a removeable pivot point means 310 positioned on first branch portion 120 of device 300. Pivot point means 310 serves as an axis around which pulling force exerted by the surgeon can be re-directed laterally towards second branch portion 130. Pivot point means 310 may be any structure capable of performing this redirection such as a ring, hook, button, or nipple. Figure 4B illustrates use of pivot means 310 in combination with distortion element 140 and connection means 150 to orient second branch portion 130 to facilitate insertion of guidewire 610. Using guidewire 610, the surgeon can optionally deploy and mate a second device (200) to second branch portion (200) using second introducer (600) as needed to extend second branch portion (130) as shown in Figure 4C.

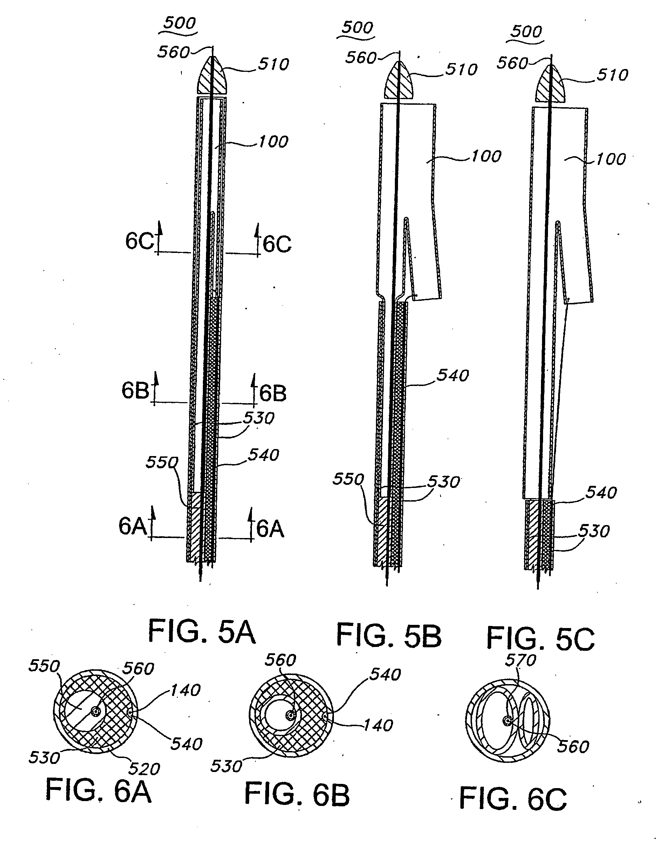

While the device 100 may be introduced in any number of ways which may be devised by those skilled in the art, one way of doing so is by the deployment apparatus illustrated in Figures 5A, 5B, 5C and 6A, 6B, and 6C.

More specifically, in Figure 5A and in Figures 6A, 6B, and 6C there is shown bifurcated endoluminal device 100 compressed for endoluminal placement in an outer sheath 520 and mounted on guidewire tube 570 through which extends guidewire 560, extending from the external access point, where the delivery apparatus enters the vasculature, to a nose cone 510 just distal (with respect to the access point) of device 100. An inner sheath 530 terminates just proximal of the proximal end of second branch portion 130 and a pusher element 550 is disposed just proximal of first branch portion 10. Distortion element 140, attached-to second branch portion 130 by removeable connection means 150, includes a return length or lengths extending within outer sheath 520 in a distortion element channel 540 to the external access point.

It will be noted that inner sheath 530 generally occupies the space within outer sheath 520 not otherwise occupied by pusher element 550 except for distortion element channel 540 which permits passage of distortion element 140.

Figure 5B illustrates deployment of bifurcated endoluminal device 100 as outer sheath 520 is retracted to the point where second branch portion 130, unbranched portion 110, and a portion of first branch portion 120 are expanded, in this case by the self expansion characteristics of their elasticity or memory metal composition. At this stage, tension can be applied to distortion element 140 (or otherwise manipulating distortion element 140), thereby constraining second branch portion 130 to a limited angular separation from first branch portion 120 and thus second branch portion 130 can be better aligned with contralateral iliac 30 (as seen in Figure 3B).

Sequentially, Figure 5C shows the final deployment stage of bifurcated endoluminal device 100 when outer sheath 520 and inner sheath 530 are both withdrawn to the point where the remainder of first branch portion 120 is deployed. As shown in Figure 3B, with distortion element 140 effecting some control over the angular disposition of second branch portion 130, capture of bifurcated endoluminal device 100 from contralateral iliac 30 can then be performed by passing guidewire/snag wire 610 from contralateral iliac 30 into open end 160. Thereafter, guidewire/snagwire 610 can be used, for example, to guide second introducer 600 for deploying and mating a leg-extending second device 200 to second branch portion 130.

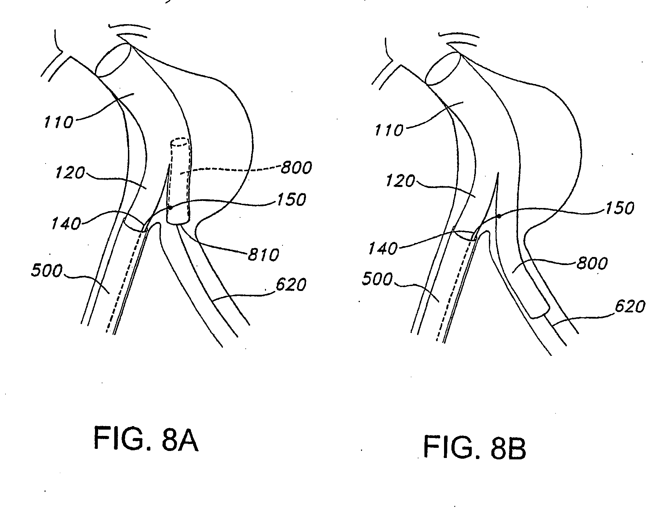

As shown in Figure 8, an optional example of bifurcated device 100 alternatively provides for the second branched portion as an everted leg (800). This device is deployed in the same manner as previous devices. However, to provide an extended second branch portion the surgeon inserts snagline 620 into open end (810) of the everted leg. As above, distortion element 140 can be used to orient open end (810) to facilitate insertion of snagline 620. Using snagline 620, the surgeon can then pull everted leg 800 down and into contralateral iliac artery 30. This example has an advantage in that in many applications would required no further leg extension of the second branch portion.

Although the invention is illustrated and described herein with reference to a specific embodiment, the invention is not intended to be limited to the details shown. The claims which follow define the scope of this invention.

## Claims

1. An endoluminal device (100) adapted to be deployed in a branched body lumen, from an access location remote from the location at which the device is to be deployed, by introduction through a first branch of the branched body lumen, said device comprising:
(a) an unbranched portion (110);
(b) two branch portions (120, 130) in communication with said unbranched portion (110), comprising
(i) a first branch portion (120) adapted to be deployed in the first branch of the body lumen, and
(ii) (ii) a second branch portion (130) adapted to be directed toward a second branch of the body lumen, and
(c) a distortion element (140) having a first end extending toward the access location and being removably connected to said second branch portion (130) **characterised in that** a single removable pivot point means (310) is positioned on an exterior portion of the first branch portion (120) such that the removable pivot point means is positioned away from an opening of the first branch portion (120), the second branch portion (130) is a different length than the first branch portion (120), and **in that** the second end of the distortion element (140) is removably connected to a single removable connection means (150) located on an exterior portion of said second branch portion away from an opening of the second branch portion (130), said distortion element (140) arranged to re-direct a tension force applied at the first end around the single removable pivot point means on the first branch portion (120) to pull the second branch portion (130) toward the first branch portion (120).

2. The endoluminal device as recited in claim 1, wherein said branched body lumen is the lower aorta and the branches thereof are the iliac arteries, and the endoluminal device is a stent graft.

3. The endoluminal device of claim 1 wherein said endoluminal device is a first device (100) and further comprising:
(d) a second device (200) adapted to be endoluminally deployed, from a second access location in a second branch lumen, said second device also adapted to be mated with the second branch portion (130) of the first device.

4. The endoluminal device of claim 1 wherein the distortion element (140) comprises an elongated filament.

5. The endoluminal device of claim 4 wherein the elongated filament is one of a wire, a yarn, and a cable.

6. The endoluminal device of claim 1 wherein the distortion element (140) is removably connected to said second branch portion (130) by a removable connection means (150) comprising one of a slipknot, a ring, and an eyelet.

## Patentansprüche

1. Endoluminale Vorrichtung (100), die dafür eingerichtet ist, von einer Zugangsstelle aus, die von der Stelle entfernt ist, an der die Vorrichtung angewendet werden soll, mittels Einführen durch eine erste Verzweigung des verzweigten Körperlumens in einem verzweigten Körperlumen angewendet zu werden, wobei die Vorrichtung Folgendes aufweist:
(a) einen nicht verzweigten Abschnitt (110);
(b) zwei Verzveigungsabschnitte (120, 130), die mit dem nicht verzweigten Abschnitt (110) in Verbindung stehen, aufweisend
(i) einen ersten Verzweigungsabschnitt (120), der dafür eingerichtet ist, in der ersten Verzweigung des Körperlumens angewendet zu werden, und
(ii)(ii) einen zweiten Verzweigungsabschnitt (130), der dafür eingerichtet ist, zu einer zweiten Verzweigung des Körperlumens geführt zu werden, und
(c) ein Verformungselement (140), das ein erstes Ende aufweist, das sich zur Zugangsstelle erstreckt und lösbar mit dem zweiten Verzweigungsabschnitt (130) verbunden ist,
**dadurch gekennzeichnet, dass** ein einzelnes lösbares Gelenkpunktmittel (310) derart an einem äußeren Abschnitt des ersten Verzweigungsabschnitts (120) platziert ist, dass das lösbare Gelenkpunktmittel abseits von einer Öffnung des ersten Verzweigungsabschnitts (120) platziert ist, der zweite Verzweigungsabschnitt (130) eine andere Länge aufweist als der erste Verzweigungsabschnitt (120), und dass das zweite Ende des Verformungselements (140) lösbar mit einem einzelnen lösbaren Verbindungsmittel (150) verbunden ist, das sich an einem äußeren Abschnitt des zweiten Verzweigungsabschnitts abseits von einer Öffnung des zweiten Verzweigungsabschnitts (130) befindet, wobei das Verformungselement (140) so angeordnet ist, dass es eine Zugkraft, die am ersten Ende aufgebracht wird, um das einzelne lösbare Gelenkpunktmittel an dem ersten Verzweigungsabschnitt (120) herum umlenkt, um den zweiten Verzweigungsabschnitt (130) zum ersten Verzweigungsabschnitt (120) hin zu ziehen.

2. Endoluminale Vorrichtung nach Anspruch 1, wobei das verzweigte Körperlumen die Bauchaorta ist und die Verzweigungen davon die Beckenarterien sind und die endoluminale Vorrichtung eine Stentprothese ist.

3. Endoluminale Vorrichtung nach Anspruch 1, wobei die endoluminale Vorrichtung eine erste Vorrichtung (100) ist und ferner Folgendes aufweist:
(d) eine zweite Vorrichtung (200), die dafür eingerichtet ist, von einer zweiten Zugangsstelle in einem zweiten Verzweigungslumen aus endoluminal angewendet zu werden, wobei die zweite Vorrichtung auch dafür eingerichtet ist, zu dem zweiten Verzweigungsabschnitt (130) der ersten Vorrichtung hinzugefügt zu werden.

4. Endoluminale Vorrichtung nach Anspruch 1, wobei das Verformungselement (140) ein längliches Filament aufweist.

5. Endoluminale Vorrichtung nach Anspruch 4, wobei das längliche Filament ein Draht, ein Faden oder eine Schnur ist.

6. Endoluminale Vorrichtung nach Anspruch 1, wobei das Verformungselement (140) über ein lösbares Verbindungsmittel (150), das einen Laufknoten, einen Ring oder eine Öse aufweist, lösbar mit dem zweiten Verzweigungsabschnitt (130) verbunden ist.

## Revendications

1. Dispositif endoluminal (100) adapté pour être déployé dans une lumière corporelle ramifiée, depuis un site d'accès éloigné du site sur lequel le dispositif doit être déployé, par l'introduction à travers une première branche de la lumière corporelle ramifiée, ledit dispositif comprenant :
(a) une partie non ramifiée (110) ;
(b) deux parties de branche (120, 130) en communication avec ladite partie non ramifiée (110), comprenant
(i) une première partie de branche (120) adaptée pour être déployée dans la première branche de la lumière corporelle, et
(ii) (ii) une seconde partie de branche (130) adaptée pour être dirigée vers une seconde branche de la lumière corporelle, et
(c) un élément de distorsion (140) ayant une première extrémité s'étendant vers le site d'accès et étant relié de façon amovible à ladite seconde partie de branche (130), **caractérisé en ce qu'**un unique moyen de point de pivot amovible (310) est positionné sur une partie extérieure de la première partie de branche (120) de telle sorte que le moyen de point de pivot amovible est positionné à distance d'une ouverture de la première partie de branche (120), la seconde partie de branche (130) présentant une longueur différente de celle de la première partie de branche (120), et **en ce que** la seconde extrémité de l'élément de distorsion (140) est reliée de façon amovible à un unique moyen de connexion amovible (150) situé sur une partie extérieure de ladite seconde partie de branche à distance d'une ouverture de la seconde partie de branche (130), ledit élément de distorsion (140) étant disposé pour rediriger une force de tension appliquée sur la première extrémité autour de l'unique moyen de point de pivot amovible sur la première partie de branche (120) pour tirer la seconde partie de branche (130) vers la première partie de branche (120).

2. Dispositif endoluminal selon la revendication 1, dans lequel ladite lumière corporelle ramifiée est l'aorte inférieure et les branches de celle-ci sont les artères iliaques, et le dispositif endoluminal est un stent greffon.

3. Dispositif endoluminal selon la revendication 1, dans lequel ledit dispositif endoluminal est un premier dispositif (100) et comprenant en outre :
(d) un second dispositif (200) adapté pour être déployé de façon endoluminale, depuis un second site d'accès dans une seconde lumière de branche, ledit second dispositif étant également adapté pour être conjugué avec la seconde partie de branche (130) du premier dispositif.

4. Dispositif endoluminal selon la revendication 1, dans lequel l'élément de distorsion (140) comprend un filament allongé.

5. Dispositif endoluminal selon la revendication 4, dans lequel le filament allongé est un fil métallique ou un fil ou un câble.

6. Dispositif endoluminal selon la revendication 1, dans lequel l'élément de distorsion (140) est relié de façon amovible à ladite seconde partie de branche (130) par un moyen de connexion amovible (150) comprenant un noeud ou un anneau ou un oeillet.
